# EUROPEAN PATENT APPLICATION

(11) **EP 4 772 527 A1**
(43) Date of publication of application: **08.07.2026**
(21) Application number: 24880052.6
(22) Date of filing: 14.10.2024
(51) Int. Cl.: C07K 7/06, A61K 8/64, A61Q 19/02, A61P 17/00, A61K 38/00

(54) **PEPTIDE HAVING SKIN WHITENING ACTIVITY AND USES THEREOF**

(30) Priority: 16.10.2023 KR 20230138102
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR); JEONG, Min Kyeong, Seoul 06188 (KR); JUNG, Da Won, Seoul 06188 (KR)
(74) Representative: Bandpay & Greuter
(86) International application number: PCT/KR2024/015493
(87) International publication number: WO 2025/084715

(57) **Abstract**

A peptide of the present invention exhibits skin whitening activity by inhibiting melanin synthesis in melanocytes and inhibiting melanosome migration. Accordingly, the peptide of the present invention can be used as an active ingredient in drugs for the treatment or prevention of hyperpigmentation diseases caused by excessive deposition of melanosomes, or as an active ingredient in cosmetics for skin whitening.

## Description

### [Technical Field]

### [Cross-citation with related applications]

This application claims the benefit of priority based on Korean Patent Application No. 10-2023-0138102 filed October 16, 2023, the entire contents of which are incorporated herein as part of this specification.

### [Technical Field]

The present invention relates to a peptide having skin whitening activity and uses thereof.

### [Background Art]

Cutaneous melanin, found in the skin, hair follicles, and eyes, plays a vital role in protecting the skin from UV rays. However, excessive melanin production can cause hyperpigmentation disorders such as melasma, freckles, and age spots, leading to psychological stress and a decline in quality of life. Melanin is synthesized mainly in melanocytes located between or below the basal layer of the epidermis and in hair follicles. It is synthesized in melanosomes, which are organelles within melanocytes. These melanosomes move to nearby keratinocytes through dendrites, and when keratinocytes rise to the outer layer, skin color is displayed. Melanin starts from L-tyrosine and is synthesized sequentially from L-tyrosine to DOPA, DOPAquinone, DOPAchrome, and DHI (5,6-dihydroxyindole) and ultimately to melanin. The reaction that produces DOPA from the L-tyrosine and DOPAquinone from DOPA is catalyzed by tyrosinase, which is known to be the most essential enzyme in melanin formation. In addition, the enzymes TRP1 (Tyrosinase-related protein 1) and TRP2 (Tyrosinase-related protein 2) are also involved in the above-mentioned melanin synthesis.

The mechanism by which melanosomes containing melanin move from melanocytes to keratinocytes is not precisely understood, but it is known that melanosomes move to the dendrites of melanocytes and are then released from them into the extracellular space, followed by membrane fusion and phagocytosis between the melanosomes and keratinocytes. Melanophilin, Rab27a, and myosin 5a proteins are known to be involved in the movement of melanosomes to the dendrites of melanocytes. Rab27a recognizes and binds to melanosomes, while melanophilin links Rab27a to myosin5a, an intracellular cytoskeletal protein. Thus, melanosomes form a complex with Rab27a, melanophilin, and myosin5a proteins, and are transported to the terminal portion of dendrites, ultimately reaching keratinocytes(J. Biochem. 2012;151(4):343-351, Lysosome-Related Organelles M Fukuda, Tohoku University, Sendai, Miyagi, Japan 2016 Elsevier Inc).

PCT International Publication No. WO2020/153819 discloses a polypeptide having an activity of inhibiting the activity of tyrosinase, a key enzyme in the melanin synthesis pathway, and its use for skin whitening. Furthermore, Korean Patent No. 10-1869783 describes a peptide having an activity that inhibits melanin production and tyrosinase activity, and its use for whitening. Previous skin whitening agent development has focused on inhibiting the activity of tyrosinase, a key enzyme in melanin synthesis. However, to achieve synergistic skin whitening effects through diverse sites of action, the development of skin whitening agents with distinct mechanisms and sites of action is necessary.

### [Prior Art Documents]

### [Patent Documents]

WO2020/153819
Korean Patent No. 10-1869783

### [Disclosure]

### [Technical Problem]

The present inventors have conducted research efforts to develop a peptide with more improved skin whitening activity, and as a result, they have experimentally demonstrated that the novel peptide developed by the present inventors not only inhibits melanin synthesis in melanocytes but also has the activity of inhibiting melanosome migration, and thus can be developed as skin whitening agents and a treatment for hyperpigmentation disorders, thereby completing the present invention.

Accordingly, one object of the present invention is to provide a novel peptide having skin whitening activity.

Another object of the present invention is to provide a composition for skin whitening comprising a peptide having the aforementioned activity as an active ingredient.

Yet another object of the present invention is to provide a pharmaceutical composition for the prevention or treatment of hyperpigmentation disorders, comprising a peptide having the aforementioned activity as an active ingredient.

Still yet another object of the present invention is to provide a cosmetic composition for skin whitening, comprising a peptide having the aforementioned activity as an active ingredient.

### [Technical Solution]

To achieve the above objects,
one aspect of the present invention provides a peptide comprising the amino acid sequence of SEQ ID NO: 1.

Another aspect of the present invention provides a composition for skin whitening comprising the peptide as an active ingredient.

Yet another aspect of the present invention provides a pharmaceutical composition for the prevention or treatment of hyperpigmentation disorders comprising the peptide as an active ingredient.

Still yet another aspect of the present invention provides a cosmetic composition for skin whitening comprising the peptide as an active ingredient.

Hereinafter, the present invention will be described in detail.

### 1. Peptide and its activity

According to one aspect of the present invention, a peptide comprising an amino acid sequence disclosed in SEQ ID NO: 1 is provided.
[Amino acid sequence of SEQ ID NO: 1]
THTISR (Thr-His-Thr-Ile-Ser-Arg)

As used herein, the term "peptide" means a linear molecule formed by amino acid residues being linked to each other by peptide bonds.

The peptide comprising the amino acid sequence of SEQ ID NO: 1 of the present invention may be used without modification, but a variant or fragment of amino acids having a different sequence by deletion, insertion, substitution, or a combination thereof of amino acid residues may be used within a range that does not affect the original activity of the peptide, for example, the activity of skin whitening.

The peptide of the present invention can be modified by phosphorylation, sulfation, acrylation, glycosylation, methylation, farnesylation, etc., within a range that does not change its activity.

The peptide of the present invention includes a peptide comprising an amino acid sequence substantially identical to a peptide comprising the amino acid sequence of SEQ ID NO: 1, and a variant or active fragment thereof. The substantially identical amino acid sequence refers to an amino acid sequence having a sequence identity of at least 75%, for example, at least 80%, at least 85%, at least 90%, at least 95%, or at least 97%, with the amino acid sequence of SEQ ID NO: 1. In addition, the peptide may additionally include a targeting sequence, a tag, a labeled residue, or an amino acid sequence manufactured for a specific purpose to increase half-life or peptide stability.

The peptide of the present invention may be modified at the N-terminus and/or C-terminus to select a portion of the amino acid sequence and enhance its activity. Such N-terminal and/or C-terminal modifications can significantly enhance the stability of the peptide of the present invention, and, for example, increase the half-life of the peptide when administered in vivo. The term "stability" above is meant to include not only stability in vivo, which protects the peptide of the present invention from attack by in vivo protein cleavage enzymes, but also storage stability (e.g., room temperature storage stability).

The N-terminal modification may be one in which a protecting group, selected from the group consisting of an acetyl group, a fluoreonylmethoxycarbonyl group, a formyl group, a palmitoyl group, a myristyl group, a stearyl group, and polyethylene glycol (PEG), is attached to the N-terminus of the peptide. The C-terminal modification may be, but is not limited to, one in which a hydroxyl group (-OH), an amino group (-NH2), an azide (-NHNH2), etc. are attached to the C-terminus of the peptide.

The peptide of the present invention can be prepared by various methods widely known in the art to which the present invention pertains. For example, the peptide of the present invention can be prepared by chemical synthesis methods known in the art, particularly solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid-phase synthesis techniques (US Patent No. 5,516,891).

The peptide of the present invention has skin whitening activity and preventive or therapeutic activity for hyperpigmentation disorders.

The peptide of the present invention has the activity of inhibiting melanin synthesis in melanocytes.

In the signaling pathway of melanin synthesis enhanced by α-MSH in melanocytes, MITF (Microphthalmia-associated transcription factor) acts as a key positive transcription factor for melanin synthesis, and CREB (cAMP-response element binding protein) is known to be a transcription factor that promotes the expression of MITF (EXPERIMENTAL AND THERAPEUTIC MEDICINE 20: 173-185, 2020).

In one embodiment, the peptide of the present invention inhibits the increase in phosphorylation levels of CREB in melanocytes under conditions in which melanin synthesis is induced (promoted).

The peptide of the present invention has the activity of inhibiting melanosome migration in melanocytes under conditions in which melanin synthesis is induced (promoted).

In one embodiment, the induction of melanin synthesis may be induced by α-MSH.

Melanosomes produced in melanocytes move to the ends of the dendrites of melanocytes, the moved melanosomes are released from the melanocytes, and the released melanosomes are incorporated into adjacent keratinocytes, ultimately being delivered to the keratinocytes. It is known that factors such as Rab27a, MYO5A (myosin VA), and MLPH (Melanophilin) are involved in the melanosome migration process in melanocytes (J. Biochem. 2012;151(4):343-351, Lysosome-Related Organelles M Fukuda, Tohoku University, Sendai, Miyagi, Japan 2016 Elsevier Inc).

In one embodiment, the peptide of the present invention has an activity of inhibiting up-regulation of the expression levels of melanosome migration related factors Rab27a, MYO5A (myosin VA), and MLPH (Melanophilin) in melanocytes under conditions in which melanin synthesis is promoted.

As described above, the peptide of the present invention can exhibit skin whitening activity and preventive or therapeutic effects on hyperpigmentation disorders through its activity of inhibiting melanin synthesis in melanocytes and inhibiting melanosome migration.

### 2. Composition for skin whitening and composition for preventing, treating or improving hyperpigmentation disorders

In another aspect of the present invention, a composition for skin whitening is provided, comprising a peptide comprising the amino acid sequence of SEQ ID NO: 1 as an active ingredient.

The peptide comprising the amino acid sequence of SEQ ID NO: 1 of the present invention has skin whitening activity through the mechanism described above.

In another aspect of the present invention, the present invention provides a pharmaceutical composition for the prevention or treatment of hyperpigmentation disorders, comprising a peptide comprising the amino acid sequence of SEQ ID NO: 1 as an active ingredient.

In one embodiment, a pharmaceutical composition comprising the peptide of the present invention as an active ingredient inhibits melanin synthesis in melanocytes or inhibits melanosome migration.

In one embodiment, a pharmaceutical composition comprising the peptide of the present invention as an active ingredient inhibits, in melanocytes under conditions in which melanin synthesis is promoted, (i) an increase in the phosphorylation level of CREB (cAMP Response Element-Binding Protein), (ii) an increase in the expression level of MITF (Microphthalmia-associated transcription factor), TYR (Tyrosinase), or TYRP1 (Tyrosinase-related protein 1), or (iii) an increase in the expression level of Rab27a, MYO5A, or MLPH (Melanophilin).

The term "hyperpigmentation disorders" in this specification refers to disorders caused by excessive increase in the amount of melanin in the skin.

Thus, skin color darkens due to an excessive increase in melanin levels. Causes of hyperpigmentation disorders include, but are not limited to, exposure to sunlight, skin inflammation (post-inflammatory hyperpigmentation) such as acne, trauma, hormonal imbalances, and medications.

In one embodiment, the hyperpigmentation disease may be melasma, freckles, age-related pigmentation, solar lentigo, or post-inflammatory hyperpigmentation of skin. The post-inflammatory hyperpigmentation of skin may be hyperpigmentation resulting from trauma, ultraviolet rays, or an inflammatory skin disease such as acne.

The pharmaceutical composition of the present invention may comprise a therapeutically effective amount of a peptide comprising the amino acid of SEQ ID NO: 1 of the present invention.

The term "therapeutically effective amount" means an amount sufficient for the peptide, which is an active ingredient of the pharmaceutical composition of the present invention, to achieve its activity or efficacy, for example, an amount sufficient to achieve the efficacy of treating or preventing hyperpigmentation disorders.

The term "prevention" as used herein means reducing the risk of developing a disease or disorder, and means any action that inhibits or delays the onset of a disease by preventing the disease or one or more of its clinical symptoms from progressing.

The term "treatment" as used herein means alleviating a disease or disorder, and includes any action that improves or beneficially alters the symptoms of a disease by arresting or reducing the progression of the disease or one or more of its clinical symptoms.

In the present invention, the prevention or treatment of hyperpigmentation disorders may be to remove the cause of hyperpigmentation in the skin or to inhibit the progression to hyperpigmentation, and specifically, to inhibit the incorporation of melanosomes into keratinocytes or to promote the decomposition of melanosomes.

The pharmaceutical composition of the present invention may include a pharmaceutically acceptable carrier.

The pharmaceutically acceptable carrier is one commonly used in formulations, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil.

The pharmaceutical composition of the present invention may additionally include, in addition to the above ingredients, a lubricant, a wetting agent, a sweetener, a flavoring agent, an emulsifier, a suspending agent, a preservative, etc., but is not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention can be administered by any suitable route for treating hyperpigmentation disorders, for example, it can be administered orally or parenterally, and in the case of parenteral administration, it can be administered by intravenous injection, subcutaneous injection, intramuscular injection, intraperitoneal injection, topical administration, transdermal administration, etc. Since the pharmaceutical composition of the present invention has an activity for preventing or treating hyperpigmentation disorders of the skin, it is preferable to apply it by topical administration, such as applying it to the skin.

The dosage of the pharmaceutical composition may be, but is not limited to, 0.0001 µg to 100 mg, 0.001 µg to 100 mg, 0.01 µg to 100 mg, 0.1 µg to 100 mg or 1.0 µg to 1000 mg per day, and may be prescribed in various ways depending on factors such as formulation method, administration method, patient age, weight, sex, pathological condition, food, administration time, administration route, excretion rate, and reaction sensitivity.

The pharmaceutical composition of the present invention can be prepared in a unit dose form or can be prepared by inserting it into a multi-dose container by formulating it using a pharmaceutically acceptable carrier and/or excipient according to a method that can be easily performed by a person having ordinary skill in the art to which the present invention pertains. In this case, the formulation may be in the form of a solution, suspension or emulsion in an oil or aqueous medium, or in the form of an extract, powder, granules, tablet or capsule, and may additionally include a dispersing agent or stabilizer.

The pharmaceutical composition of the present invention may be a topical skin preparation. The topical skin preparation is a preparation that can be applied to the outside of the skin. When the pharmaceutical composition of the present invention is used as a topical skin preparation, it may be applied to the skin, specifically to an area of the skin where hyperpigmentation has occurred. The topical skin preparation may be a cream, gel, ointment, skin emulsifier, skin suspension, transdermal patch, drug-containing bandage, lotion, or a combination thereof. The topical skin preparation may be appropriately mixed with ingredients commonly used in topical skin preparations such as cosmetics or medicines, for example, aqueous ingredients, oily ingredients, powder ingredients, alcohols, moisturizers, thickeners, UV absorbers, whitening agents, preservatives, antioxidants, surfactants, fragrances, colorants, various skin nutrients, or a combination thereof, as needed. The topical skin preparation may also appropriately contain metal sequestrants such as sodium edetate, trisodium edetate, sodium citrate, sodium polyphosphate, sodium metaphosphate, and gluconic acid; caffeine, tannin, verapamil, licorice extract, glabridin, hot water extract of the fruit of *Pseudocydonia sinensis*; various herbal medicines; tocopheryl acetate, glycyrrhizic acid, tranexamic acid and derivatives or salts thereof; and vitamin C, magnesium ascorbic acid phosphate, ascorbic acid glucoside, arbutin, kojic acid, and sugars such as glucose, fructose and trehalose.

In another aspect of the present invention, the present invention provides cosmetic compositions for skin whitening comprising a peptide comprising the amino acid sequence of SEQ ID NO: 1 as an active ingredient.

In one embodiment, a cosmetic composition comprising the peptide of the present invention as an active ingredient inhibits melanin synthesis in melanocytes or inhibits melanosome migration.

In one embodiment, a cosmetic composition comprising the peptide of the present invention as an active ingredient inhibits, in melanocytes under conditions in which melanin synthesis is induced, (i) an increase in the phosphorylation level of CREB (cAMP Response Element-Binding Protein), (ii) an increase in the expression level of MITF (Microphthalmia-associated transcription factor), TYR (Tyrosinase), or TYRP1 (Tyrosinase-related protein 1), or (iii) an increase in the expression level of Rab27a, MYO5A, or MLPH (Melanophilin).

The cosmetic composition may be prepared in any formulation commonly prepared in the technical field to which the present invention pertains, and may be a topical skin preparation. For example, it may be formulated as a solution, suspension, emulsion, paste, gel, cream, lotion, powder, soap, surfactant-containing cleanser, oil, powder foundation, emulsion foundation, wax foundation, and spray, but is not limited thereto.

The cosmetic composition may be prepared in various forms such as a solution, sol-gel, emulsion, oil, wax, aerosol, etc., such as a flexible toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, a gel, etc., but is not limited thereto.

The cosmetic composition of the present invention may include other additives such as excipients and carriers, and it is possible to apply and mix as much as necessary the usual ingredients mixed in general skin cosmetics.

When the formulation of the cosmetic composition is in the form of a paste, cream or gel, as a carrier component, animal oil, vegetable oil, wax, paraffin, starch, tragacanth, cellulose derivatives, polyethylene glycol, silicone, bentonite, silica, talc or zinc oxide may be used.

When the formulation of the cosmetic composition is in the form of a powder or spray, as a carrier component, lactose, talc, silica, aluminum hydroxide, calcium silicate or polyamide powder may be used. In particular, in the case of a spray, a propellant such as chlorofluorohydrocarbon, propane/butane or dimethyl ether may be additionally included, but is not limited thereto.

When the formulation of the cosmetic composition is in the form of a solution or emulsion, a solvent, a solubilizer or an emulsifier may be used as a carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan.

When the formulation of the cosmetic composition is in the form of a suspension, a liquid diluent such as water, ethanol or propylene glycol, a suspending agent such as ethoxylated isostearyl alcohol, polyoxyethyl sorbitol ester and polyoxyethylene sorbitan ester, microcrystalline cellulose, aluminum metahydroxide, bentonite, agar or tragacanth, etc. can be used as a carrier component.

When the formulation of the cosmetic composition is a surfactant-containing cleansing, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, fatty alcohol, fatty acid glyceride, fatty acid diethanolamide, vegetable oil, lanolin derivative, or ethoxylated glycerol fatty acid ester may be used as a carrier component.

When the formulation of the cosmetic composition is a hair shampoo, base ingredients for forming a shampoo, such as a thickener, a surfactant, a viscosity modifier, a moisturizer, a pH adjuster, a preservative, and essential oils, may be mixed. CDE may be used as the thickener, LES, which is an anionic surfactant, and coco betaine, which is an amphoteric surfactant, may be used as the surfactant, polyquaternary may be used as the viscosity modifier, glycerin may be used as the moisturizer, and citric acid or sodium hydroxide may be used as the pH adjuster. As the preservative, grapefruit extract, etc. can be used, and in addition, essential oils such as cedarwood, peppermint, and rosemary, as well as silk amino acids, pentaol, or vitamin E can be added.

The ingredients included in the cosmetic composition may further include, in addition to the peptide of the present invention as an active ingredient and the carrier component, ingredients commonly used in cosmetic compositions, such as conventional auxiliary agents such as antioxidants, stabilizers, solubilizers, vitamins, pigments, and fragrances, but are not limited thereto.

The peptide of the present invention may be included in the above-described composition, pharmaceutical composition or cosmetic composition at a concentration of 0.01 µM to 1000 µM, specifically, the peptide of the present invention may be included at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM , but is not limited thereto.

### 3. Use of peptide of the present invention

In another aspect of the present invention, a peptide comprising the amino acid sequence of SEQ ID NO: 1 is provided for use in skin whitening or in preventing, treating or improving hyperpigmentation disorders.

In another aspect of the present invention, a skin whitening method is provided, comprising administering a peptide comprising the amino acid sequence of SEQ ID NO: 1 or a composition comprising the peptide to a subject in need of skin whitening.

In another aspect of the present invention, a method for preventing or treating hyperpigmentation disorders is provided, comprising administering a peptide comprising the amino acid sequence of SEQ ID NO: 1 or a composition comprising the peptide to a subject in need of prevention or treatment of hyperpigmentation disorders.

In another aspect of the present invention, there is provided the use of a peptide comprising the amino acid sequence of SEQ ID NO: 1 for the preparation of a medicament for the prevention or treatment of hyperpigmentation disorders.

In another aspect of the present invention, the use of a peptide comprising the amino acid sequence of SEQ ID NO: 1 for the manufacture of cosmetics for skin whitening is provided.

### [Advantageous Effects]

The peptide of the present invention exhibits skin whitening activity by inhibiting melanin synthesis and melanosome migration in melanocytes. Therefore, the peptide of the present invention can be used as an active ingredient in drugs for the treatment or prevention of hyperpigmentation disorders caused by excessive deposition of melanosomes, or as an effective ingredient in cosmetics for skin whitening.

However, the effects of the present invention are not limited to the effects mentioned above, and other effects not mentioned will be clearly understood by those skilled in the art from the description below.

### [Description of Drawings]

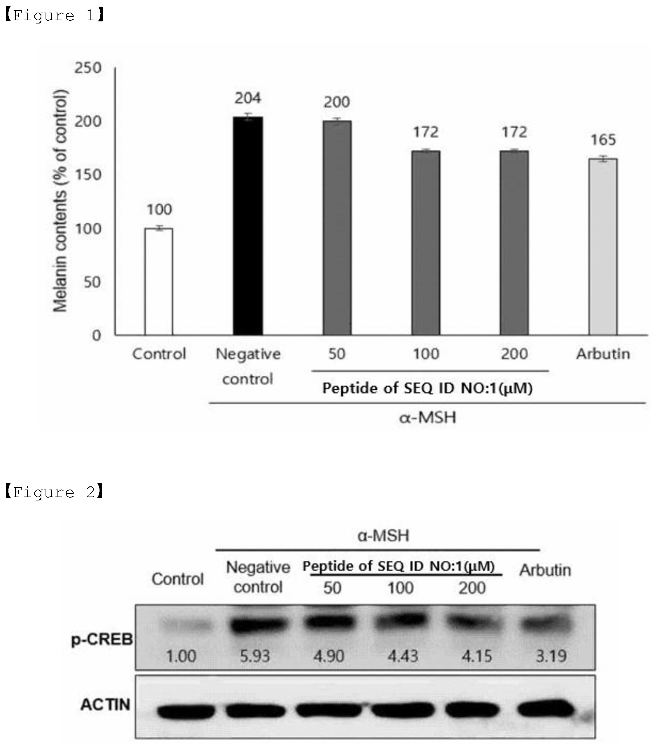

FIG. 1 shows the results of measuring melanin content after treating B16F10 cells with α-MSH, the peptide of Preparation Example 1, and arbutin.
FIG. 2 shows the results of determining the phosphorylation level of CREB after treating B16F10 cells with α-MSH, the peptide of Preparation Example 1, and arbutin. The band values are calculated by normalizing the densitometric intensity of the p-CREB band to that of the actin band, which is the loading control, and represented as relative expression levels compared to the control set as 1.

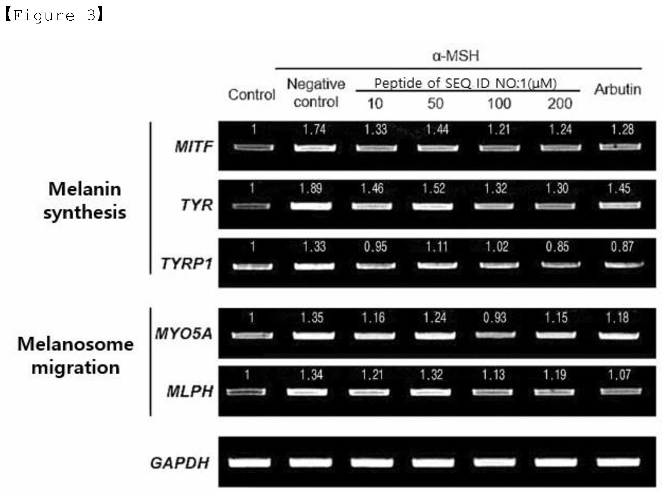

FIG. 3 shows the results of determining the expression levels of genes of MITF, TYR, TYRP1, which are factors related to melanin production, and MYO5A, MLPH, which are factors related to melanosome movement, by RT-PCR after treating B16F10 cells with α-MSH, the peptide of Preparation Example 1, and arbutin. The band values are calculated by normalizing the densitometric intensity of each marker's band to that of the GAPDH band, which is a loading control, and represented as relative expression levels compared to the control set as 1.

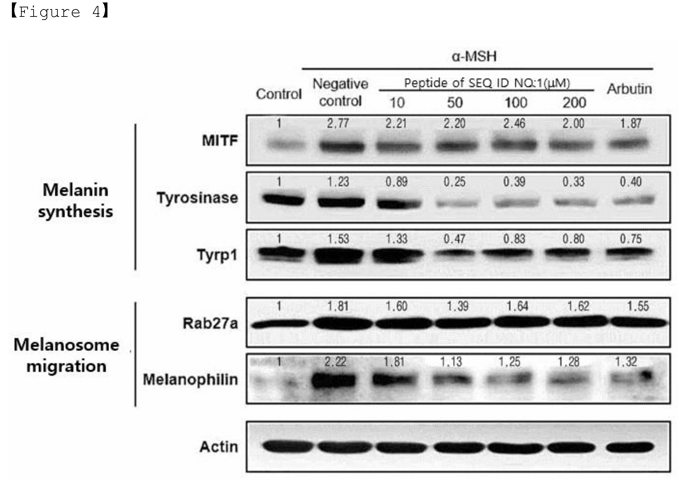

FIG. 4 shows the results of Western blot analysis of the expression levels of MITF, Tyrosinase, and TYRP1, which are factors related to melanin production, and Rab27a and Melanophilin, which are factors related to melanosome migration, after treating B16F10 cells with α-MSH, the peptide of Preparation Example 1, and arbutin. The band values are calculated by normalizing the densitometric intensity of each marker's band to that of the Actin band, which is a loading control, and represented as relative expression level compared to the control set as 1.

### [Mode For Carrying Out The Invention]

Hereinafter, the present invention will be described in detail by way of examples. However, the following examples specifically illustrate the present invention, and the content of the present invention is not limited by the following examples.

### Preparation Example 1: Preparation of peptide

A peptide having the amino acid sequence of SEQ ID NO: 1 shown in Table 1 below was synthesized using an automatic peptide synthesizer (Milligen 9050, Millipore, USA), and the synthesized peptide was purified using C18 reverse-phase high-performance liquid chromatography (HPLC) (Waters Associates, USA). The column used was ACQUITY UPLC BEH300 C18 (2.1 mm X 100 mm, 1.7 µm, Waters Co, USA).

**[Table 1]**

| SEQ ID NO | Amino acid sequence of peptide |
|---|---|
| 1 | THTISR (Thr-His-Thr-Ile-Ser-Arg) |

The efficacy of the peptide of SEQ ID NO: 1 prepared above was evaluated through the following experiment.

### Experimental Example 1: Analysis of melanin content

It was confirmed whether the peptide of SEQ ID NO: 1 prepared in Preparation Example 1 above inhibits melanin synthesis in melanocytes.

B16F10 mouse melanoma cells were seeded at a density of 7 x 10⁴ cells/well in 6-well cell culture plates and cultured for 24 hours. Subsequently, the cells were replaced with culture medium containing 2% FBS, and then treated with 200 ng/mL α-MSH (α-Melanocyte-stimulating hormone) and the peptide prepared in Preparation Example 1, followed by incubation for 72 hours. At this time, the positive control was treated with 200 ng/mL α-MSH and 500 µM arbutin. After harvesting the cultured cells, cell lysate was prepared by treating with 1 N NaOH. The absorbance at a wavelength of 450 nm was measured using a spectrophotometer.

As a result of the experiment, as can be seen from the melanin content analysis results in FIG. 1, it was confirmed that the melanin content in B16F10 cells increased by α-MSH treatment, and the increased melanin content decreased in a concentration-dependent manner by treatment with the peptide of Preparation Example 1.

### Experimental Example 2: Analysis of the expression regulation of MITF, a transcription factor involved in melanogenesis.

The effect of the peptide of SEQ ID NO: 1 prepared in Preparation Example 1 above on the regulation of expression of MITF (Microphthalmia-associated transcription factor), which is a transcription factor for melanin synthesis, in melanocytes under conditions where melanin synthesis is promoted was confirmed by measuring the phosphorylation level of CREB (cAMP Response Element-Binding Protein). In the signaling pathway of α-MSH-induced melanin synthesis in melanocytes, CREB is known to act as a transcription factor for MITF and participate in regulating its expression (EXPERIMENTAL AND THERAPEUTIC MEDICINE 20: 173-185, 2020).

B16F10 mouse melanoma cells were seeded at a density of 3 x 10⁵ cells/well in 6-well cell culture plates and cultured for 24 hours. The medium was then replaced with serum-free medium and incubated for 24 hours. 200 ng/mL α-MSH and the peptide from Preparation Example 1 were treated and incubated for 10 minutes. At this time, the positive control group was treated with 200 ng/mL α-MSH and 500 µM arbutin. After washing with PBS, cell lysate was prepared by treating with lysis buffer, and BCA was quantified to prepare an equal amount of protein sample. Electrophoresis was performed on the prepared cell lysate and protein samples using a 10% SDS-PAGE gel. The proteins separated by SDS PAGE were transferred to a PVDF membrane and blocked with 5% skim milk for 30 minutes at room temperature. Primary antibodies against p-CREB (Cell Signaling, USA) and actin (Santa Cruz, USA) were diluted in 3% BSA at a ratio of 1:1000 and reacted with the PVDF membrane at 4°C for 16 hours. The membrane was washed three times for 15 minutes each with 0.1% PBS-T (0.1% Tween-20 in PBS). Subsequently, secondary antibodies (Jackson ImmunoResearch, USA) were diluted in 5% skim milk at a ratio of 1:2000 and reacted at room temperature for 1 hour. The cells were washed three times for 15 minutes each with 0.1% PBS-T (0.1% Tween-20 in PBS). After treatment with ECL solution (GE Healthcare, USA), the expression level was analyzed using ImageQuant 800 (Cytiva, USA).

As a result of the experiment, as can be seen from the results of the analysis of the phosphorylation level of CREB in FIG. 2, it was confirmed that the phosphorylation level of CREB increased by α-MSH treatment, and the increased phosphorylation level of CREB decreased in a concentration-dependent manner by treatment with the peptide of Preparation Example 1.

### Experimental Example 3: Expression analysis of factors involved in melanin formation and melanosome migration - mRNA expression analysis using RT-PCR

The effect of the peptide of SEQ ID NO: 1 prepared in Preparation Example 1 above on the expression of factors related to melanin synthesis in melanocytes, MITF (Microphthalmia-associated transcription factor), TYR (Tyrosinase), TYRP1 (Tyrosinase-related protein 1), and factors related to melanosome migration, MYO5A (myosin VA) and MLPH (Melanophilin), was confirmed by measuring the mRNA expression level using RT-PCR.

B16F10 mouse melanoma cells were seeded at a density of 5 x 10⁴ cells/well in 6-well cell culture plates and cultured for 24 hours. Subsequently, the medium was replaced with culture medium containing 2% FBS, and then 200 ng/mL α-MSH and the peptide of Preparation Example 1 were treated, followed by incubation for 72 hours. At this time, the positive control group was treated with 200 ng/mL α-MSH and 500 µM arbutin. After washing with PBS, RNA was isolated by treating with 300 µL of easy blue (intron, Korea). The isolated RNA was quantified and cDNA synthesis was performed using a cDNA synthesis kit (enzynomics, Korea). PCR was performed using the primers in Table 2 and PCR premix (enzynomics, Korea). The PCR reaction products were electrophoresed on a 1.5% agarose gel. Bands were measured using a Bio-Rad gel image system.

**[Table 2]**

| Primer name | Sequence (5' → 3') | SEQ ID NO |
|---|---|---|
| MITF Forward | (5') CCAGCCTGGCGATCATGTCAT (3') | 2 |
| MITF Reverse | (5') GGTCTGGACAGGAGTTGCTG (3') | 3 |
| TYR Forward | (5') GGCCAGCTTTCAGGCAGAGG (3') | 4 |
| TYR Reverse | (5') TGGTGCTTCATGGGCAAAAT (3') | 5 |
| TYRP1 Forward | (5') TCTGTGAAGGTGTGCAGGAG (3') | 6 |
| TYRP1 Reverse | (5') CCGAAACAGAGTGGAAGGTT (3') | 7 |
| MYO5A Forward | (5') TTCTACATTGTGGGCGCCAT (3') | 8 |
| MYO5A Reverse | (5') TCCTCCAGGTTGGTCAATCG (3') | 9 |
| MLPH Forward | (5') ACGATGTCAGGGGCAAACAT (3') | 10 |
| MLPH Reverse | (5') CTCCTCTGTGTCAGCACTGG (3') | 11 |
| GAPDH Forward | (5') GGTGTGAACGGATTTGGCCGTATTG (3') | 12 |
| GAPDH Reverse | (5') CCGTTGAATTTGCCGTGAGTGGAGT (3') | 13 |

As a result of the experiment, as can be seen from the results in FIG. 3, it was confirmed that the expression of MITF, TYR, and TYRP1 genes, which are factors related to melanin production, and MYO5A and MLPH genes, which are factors related to melanosome migration, increased by α-MSH treatment, and the increased expression levels of the genes decreased again by treatment with the peptide of Preparation Example 1.

### Experimental Example 4: Expression analysis of factors involved in melanin formation and melanosome migration - protein expression analysis using Western blot

The effect of the peptide of SEQ ID NO: 1 prepared in Preparation Example 1 above on the expression of factors related to melanin synthesis in melanocytes, such as MITF (Microphthalmia-associated transcription factor), tyrosinase (TYR), TYRP1 (Tyrosinase-related protein 1), and factors related to melanosome migration, such as Rab27a and melanophilin, was confirmed by measuring the protein expression level using Western blot.

B16F10 mouse melanoma cells were seeded at a density of 7 x 10⁴ cells/well in 6-well cell culture plates and cultured for 24 hours. Subsequently, the medium was replaced with culture medium containing 2% FBS, and then 200 ng/mL α-MSH and the peptide of Preparation Example 1 were treated, followed by incubation for 72 hours. At this time, the positive control group was treated with 200 ng/mL α-MSH and 500 µM arbutin. After washing with PBS, cell lysate was prepared by treating with lysis buffer, and the same amount of protein sample was prepared by BCA quantitation. Electrophoresis was performed on the prepared cell lysate and protein sample using 10% SDS-PAGE gel. The proteins separated through SDS PAGE were transferred to a PVDF membrane and blocked using 5% skim milk at room temperature for 30 minutes. Primary antibodies against MITF, TYR, TYRP1, Rab27a, and melanophilin (Cell signaling, USA) and primary antibodies against actin (Santa Cruz, USA) were diluted in 3% BSA at a ratio of 1:1000 and reacted with PVDF membranes at 4°C for 16 hours. The membranes were washed three times for 15 minutes each with 0.1% PBS-T (0.1% Tween-20 in PBS). Subsequently, secondary antibodies (Jackson ImmunoResearch, USA) were diluted in 5% skim milk at a ratio of 1:2000 and reacted for 1 hour at room temperature. The membranes were washed three times for 15 minutes each with 0.1% PBS-T (0.1% Tween-20 in PBS). After treatment with ECL solution (GE Healthcare, USA), the expression level was analyzed using ImageQuant 800 (Cytiva, USA) equipment.

As a result of the experiment, as can be seen from the results in FIG. 4, it was confirmed that the expression of MITF, TYR, and TYRP1 proteins, which are factors related to melanin production, and Rab27a and Melanophilin proteins, which are factors related to melanosome migration, increased by α-MSH treatment, and the increased expression levels of the above proteins decreased again by treatment with the peptide of Preparation Example 1.

Although representative embodiments of the present application have been described above as examples, the scope of the present application is not limited only to the specific embodiments described above, and a person with ordinary knowledge in the relevant field will be able to make appropriate changes within the scope described in the claims of the present application.

## Claims

1. A peptide comprising an amino acid sequence of SEQ ID NO: 1.

2. A composition for skin whitening comprising the peptide of claim 1 as an active ingredient.

3. A pharmaceutical composition for the prevention or treatment of hyperpigmentation disorders, comprising the peptide of claim 1 as an active ingredient.

4. The pharmaceutical composition for the prevention or treatment of hyperpigmentation disorders of claim 3, wherein the peptide inhibits (i) melanin synthesis or (ii) melanosome migration in melanocytes.

5. The pharmaceutical composition for the prevention or treatment of hyperpigmentation disorders of claim 4, wherein the peptide has the activity of:
(i) inhibiting an increase in the phosphorylation level of CREB (cAMP Response Element-Binding Protein),
(ii) inhibiting an increase in the expression level of MITF (Microphthalmia-associated transcription factor), TYR (Tyrosinase), or TYRP1 (Tyrosinase-related protein 1), or
(iii) inhibiting an increase in the expression level of Rab27a, MYO5A, or MLPH (Melanophilin) in melanocytes where melanin synthesis is induced.

6. The pharmaceutical composition for the prevention or treatment of hyperpigmentation disorders of claim 3, wherein the hyperpigmentation disorders are disorders that occur when the amount of melanin in the skin increases excessively.

7. The pharmaceutical composition for the prevention or treatment of hyperpigmentation disorders of claim 3, wherein the hyperpigmentation disorders are melasma, freckles, age-related pigmentation, solar lentigo, or post-inflammatory hyperpigmentation of skin.

8. The pharmaceutical composition for the prevention or treatment of hyperpigmentation disorders of claim 3, wherein the pharmaceutical composition is a topical skin preparation.

9. A cosmetic composition for skin whitening comprising the peptide of claim 1 as an active ingredient.

10. The cosmetic composition for skin whitening of claim 9, wherein the peptide inhibits (i) melanin synthesis or (ii) melanosome migration in melanocytes.

11. The cosmetic composition for skin whitening of claim 10, wherein the peptide has the activity of:
(i) inhibiting an increase in the phosphorylation level of CREB (cAMP Response Element-Binding Protein),
(ii) inhibiting an increase in the expression level of MITF (Microphthalmia-associated transcription factor), TYR (Tyrosinase), or TYRP1 (Tyrosinase-related protein 1), or
(iii) inhibiting an increase in the expression level of Rab27a, MYO5A, or MLPH (Melanophilin) in melanocytes where melanin synthesis is induced.

12. The cosmetic composition for skin whitening of claim 9, wherein the cosmetic composition is a topical skin preparation.

13. The cosmetic composition for skin whitening of claim 12, wherein the cosmetic composition is at least one formulation selected from the group consisting of a solution, a suspension, an emulsion, a paste, a gel, a cream, a lotion, a powder, a soap, a surfactant-containing cleanser, an oil, a powder foundation, an emulsion foundation, a wax foundation, and a spray.

14. A method for preventing or treating hyperpigmentation disorders, comprising administering the peptide of claim 1 or a composition comprising the peptide to a subject in need of prevention or treatment of hyperpigmentation disorders.

15. The method for preventing or treating hyperpigmentation disorders of claim 14, wherein the hyperpigmentation disorders are melasma, freckles, age-related pigmentation, solar lentigo, or post-inflammatory hyperpigmentation of skin.
